(19) Europäisches Patentamt
European Patent Office
Office européen des brevets

(11) Veröffentlichungsnummer : **0 444 506 B1**

(12) # EUROPÄISCHE PATENTSCHRIFT

(45) Veröffentlichungstag der Patentschrift : **26.10.94 Patentblatt 94/43**

(21) Anmeldenummer : **91102391.9**

(22) Anmeldetag : **20.02.91**

(51) Int. Cl.[5] : **G03C 1/10,** C07D 213/20, C07D 213/34, C07D 213/65, C07D 213/73, C07D 213/82, C07D 401/04, C07D 215/10, C07D 217/10, C07D 233/54, C07D 213/38

(54) **Arylhydrazide enthaltende photographische Silberhalogenidmaterialien.**

(30) Priorität : **26.02.90 DE 4006032**

(43) Veröffentlichungstag der Anmeldung : **04.09.91 Patentblatt 91/36**

(45) Bekanntmachung des Hinweises auf die Patenterteilung : **26.10.94 Patentblatt 94/43**

(84) Benannte Vertragsstaaten : **BE CH DE ES FR GB IT LI NL SE**

(56) Entgegenhaltungen :
**DE-C- 3 829 078**
**PATENT ABSTRACTS OF JAPAN, Band 12, Nr. 161 (P-702)[3008] 17. Mai 1988, & JP-A-62 275247 (MITSUBISHI PAPER MILLS LTD.) 30. November 1987**
**RESEARCH DISCLOSURE, Nr. 235, November 1983, HAVANT, GB; Seiten 346-352; Anonymous: "Development nucleation by hydrazine and hydrazine derivatives"**

(73) Patentinhaber : **DU PONT DE NEMOURS (DEUTSCHLAND) GMBH**
**Du Pont Strasse 1**
**D-61352 Bad Homburg (DE)**
(84) **CH DE ES LI NL SE**
Patentinhaber : **E.I. DU PONT DE NEMOURS AND COMPANY**
**1007 Market Street**
**Wilmington Delaware 19898 (US)**
(84) **BE FR GB IT**

(72) Erfinder : **Rüger, Reinhold, Dr.**
**Schillerstrasse 11**
**W-6074 Rödermark (DE)**
Erfinder : **Chan, Dominic M. T., Dr.**
**1506 Delwood Road**
**Wilmington, Delaware 19803 (US)**

(74) Vertreter : **Pistor, Wolfgang**
**Du Pont de Nemours (Deutschland) GmbH**
**Patentabteilung**
**Dornhofstrasse 10**
**D-63263 Neu-Isenburg (DE)**

**EP 0 444 506 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft photographische Silberhalogenidmaterialien zur Erzeugung von Bildern mit ultrasteiler Gradation vorzugsweise für die Schnellverarbeitung, die bestimmte Arylhydrazide enthalten, sowie neue Arylhydrazide für die Verwendung in solchen photographischen Materialien.

Photographische Silberhalogenidsysteme mit ultrasteiler Gradation werden beispielsweise in der Reproduktionstechnik zum Erzeugen von gerasterten Bildern aus Halbtonaufzeichnungen, für die Fotosatztechnik, sowie für Strichaufnahmen und Fotomasken verwendet. Der Begriff "ultrasteil" soll dabei darstellen, daß die Gradation höher ist als man erwarten kann, wenn man annimmt, daß jedes einzelne Emulsionskorn unabhängig von seinen Nachbarn belichtet und entwickelt wird. Solche Systeme nutzen beispielsweise Effekte aus, bei denen durch die Entwicklung eines Korns die Entwicklung benachbarter Körner initiiert wird, auch wenn diese nicht hinreichend belichtet wurden um für sich allein entwickelbar zu sein ("ansteckende Entwicklung").

Seit langem bekannt sind sogenannte Lith-Systeme. Diese bestehen aus Filmen, bei denen meist der größte Anteil des Silberhalogenids als Chlorid vorliegt und dazugehörigen Entwicklern, welche durch einen relativ hohen pH-Wert, einen niedrigen Gehalt an Sulfit und das Fehlen superadditiv wirkender Entwicklersubstanzen gekennzeichnet sind. Dementsprechend ist die Lichtempfindlichkeit der Filme und ihre Entwicklungsgeschwindigkeit relativ begrenzt und es erfordert einen beträchtlichen Aufwand, die Aktivität der Entwickler über längere Zeit konstant zu halten.

Diese Nachteile sind durch neuerdings in die Praxis eingeführte Systeme gemildert worden, bei denen die Entwicklung des photographischen Materials in Gegenwart gewisser Hydrazinverbindungen durchgeführtwird. Eine Zusammenfassung der umfangreichen Literatur hierzu gibt die Research Disclosure 235 010 (November 1983). Danach werden meist sogenannte aktivierte Hydrazinverbindungen verwendet, die durch die allgemeine Formel CT - N $R^1$ - N $R^2$ - Ac beschrieben werden können. Dabei bedeutet CT einen tertiären Kohlenstoff - meist als Bestandteil eines aromatischen Ringsystems wie Phenyl-, $R^1$ und $R^2$ alkalisch abspaltbare Reste und Ac eine aktivierende Gruppe. Die Hydrazinverbindungen werden üblicherweise den lichtempfindlichen Schichtsystemen zugesetzt. Durch die Einwirkung der alkalischen Entwicklerlösung im Zusammenwirken mit den bei der Entwicklung der Silberhalogenidkörner aus der Entwicklersubstanz entstehenden Oxidationsprodukten sollen dann freie Hydrazinverbindungen entstehen, die benachbarte Körner verschleiern. In der Praxis bevorzugt sind Hydrazinverbindungen, bei denen die aktivierenden Gruppen über Carbonylgruppen an den Hydrazinstickstoff gebunden sind. Wenn es sich bei CT um einen tertiären Kohlenstoff in einer aromatischen Gruppe handelt, werden diese Substanzen auch als Arylhydrazide bezeichnet.

Ein Nachteil der Systeme mit Hydrazinverbindungen besteht darin, daß die Entwicklung bei relativ hohen pH-Werten durchgeführt werden muß. Die einschlägigen Druckschriften beschreiben zwar Entwickler-pH-Werte im Bereich von etwa 9 bis 12,5, in der Praxis wird jedoch ausschließlich bei Werten über 11,5 gearbeitet, weil anders keine befriedigende Entwicklungsgeschwindigkeit erreicht wird und die Bildqualität ungenügend ist. Daher sind die Entwicklerlösungen auch nicht für ein problemfreies Arbeiten hinreichend stabil. Sie sind insbesondere trotz ihrer hohen Sulfitgehalte sehr empfindlich gegenüber Luftsauerstoff. Auch wird durch unvermeidliche geringe Schwankungen des pH-Wertes während des Betriebs die Entwicklungscharakteristik so stark verändert, daß es schwierig ist, über längere Zeit gleichmäßige Ergebnisse zu erhalten. Weitere Probleme sind die starke Korrosion der Entwicklungsmaschinen durch die hochalkalischen Entwicklerlösungen sowie die Entsorgung der vergleichsweise stark gepufferten verbrauchten Lösungen.

In der EP 02 53 665-A1 werden photographische Materialien vorgeschlagen, die Hydrazinverbindungen enthalten, bei denen die aktivierende Gruppe im alkalischen Entwicklermedium unter Ausbildung einer ringförmigen Struktur abgespalten wird. Diese Materialien lassen sich schon bei pH 11 mit befriedigendem Resultat entwickeln. Hierdurch werden die oben geschilderten Nachteile zwar gemildert; es besteht aber nach wie vor ein Bedürfnis für eine weitere Verbesserung. Darüber hinaus sind die dort verwendeten Arylhydrazide nur über mehrstufige Synthesen bzw. mit mäßigen Ausbeuten herstellbar.

Obgleich sich die Hydrazinverbindungen schon heute in vieler Hinsicht den Lithsystemen technisch überlegen zeigen, besteht doch insbesondere das Bedürfnis, den Verarbeitungsprozeß, dessen Dauer maßgeblich durch die Entwicklungszeit bestimmt wird, noch weiter zu beschleunigen.

Aufgabe der Erfindung ist es daher, photographische Silberhalogenidmaterialien mit Hydrazinverbindungen anzugeben, die sich bei relativ niedrigen pH-Werten vergleichsweise schnell zu ultrasteiler Gradation entwickeln lassen. Eine weitere Aufgabe ist es, Materialien dieser Art anzugeben, bei denen das Ergebnis der Entwicklung nur wenig vom pH-Wert des Entwicklers abhängt. Eine weitere Aufgabe besteht darin, neue Hydrazinverbindungen anzugeben, die zur Herstellung von solchen Materialien geeignet sind und mit geringem Aufwand und guter Ausbeute hergestellt werden können.

Diese Aufgabe wird gelöst durch photographische Silberhalogenidmaterialen, die Verbindungen der Formel (I) enthalten,

$$Ar - NR - NR^1 - G - X^+ A^- \qquad (I)$$

worin
Ar eine substituierte Phenylgruppe oder eine andere substituierte oder unsubstituierte Arylgruppe,
G die Gruppe CO, SO, $SO_2$, Phosphonyl, Phosphoryl oder C=$NR^2$,
$X^+$ einen Rest, der eine kationische Gruppe mit einem quaternierten Stickstoffatom enthält,
R, $R^1$, $R^2$ Wasserstoff, Alkyl oder Alkylsulfinyl mit 1 bis 6 Kohlenstoffatomen,
$A^-$ ein Anion bedeuten,
dadurch gekennzeichnet, daß
das quaternierte Stickstoffatom in eine substituierte oder unsubstituierte Imidazolium-, Imidazolinium-, Isochinolinium-, Chinoliniumgruppe oder in eine an einem oder mehreren Kohlenstoffatomen substituierte Pyridiniumgruppe, ausgenommen Sulfoethylpyridinium, eingebunden ist.

Der Rest Ar kann außer durch einen substituierte Phenylrest auch durch einen anderen, substituierten oder unsubstituierten Arylrest, beispielsweise einen Naphthyl-, einen Anthryl- oder einen Phenanthrylrest dargestellt werden.

Die Substituenten am aromatischen Ringsystem des Restes Ar enthalten bevorzugt solche Gruppen, die gemäß dem Stand der Technik angewendet werden, um der Hydrazinverbindung bestimmte Eigenschaften, wie z.B. eine bestimmte Diffusionsfähigkeit (Ballastgruppen) oder ein bestimmtes Adsorptionsverhalten am Silberhalogenid (adsorptionsfördernde Gruppen), zu verleihen. Beispiele solcher Substituenten sind unverzweigtes, verzweigtes oder cyclisches Alkyl, Alkenyl oder Alkinyl, vorzugsweise mit 1 bis 20 Kohlenstoffatomen, das seinerseits auch weiter mit einem der in diesem Absatz genannten Reste substituiert sein kann, Halogenatome, Cyan, Carboxyl, Amino, substituierte oder unsubstituierte Arylreste mit 6 bis 14 Kohlenstoffatomen, Alkylamino- und Acylaminoreste mit 1 bis 20 Kohlenstoffatomen, Thioharnstoffreste und andere Thiocarbonylgruppen enthaltende Reste, Alkoxy- und Aryloxyreste, aliphatische und aromatische Acyloxyreste, Urethangruppen, Alkyl- und Arylsulfonyl, Alkyl- und Arylsulfonamidoreste sowie Reste stickstoff- oder schwefelhaltiger Heterocyclen mit 5 bis 10 Gliedern, wie Imidazol, Thiazol, Benzthiazol, Benzimidazol. Die genannten Substituenten können unabhängig voneinander an den Arylrest gebunden oder auch, sich gegenseitig substuierend, zu einer Kette verbunden sein. Günstig wirken solche Substituenten, welche die Elektronendichte des aromatischen Ringsystems durch mesomere oder induktive Effekte erhöhen.

Daß die erfindungsgemäßen Verbindungen mit kationischen Gruppen im aktivierenden Rest verbesserte Eigenschaften, insbesondere eine höhere Entwicklungsgeschwindigkeit bei geringen pH-Werten, besitzen würden, war vom Fachmann aufgrund des Standes der Technik nicht vorauszusehen. Ein bekannter Vergleichsversuch mit einfachen Hydrazinen (DE 27 25 743 C3, Seite 14) zeigt vielmehr keinerlei Einfluß einer solchen Gruppe auf die Entwicklung der Gradation. Nach DE 11 99 612 wirkt ein am Arylrest unsubstituiertes Arylhydrazid mit einer kationischen Gruppe im Acylrest auf hochempfindliche Jodobromidemulsionen stark schleiernd ohne daß die Gradation beeinflußt wird. Dagegen zeigen die erfindungsgemäßen Materialien auch bei längerer Lagerung keinen Schleieranstieg und bei geeigneter Entwicklung auch ultrasteile Gradation.

Das Anion $A^-$ kann ein Halogenidanion, beispielsweise ein Chlorid-, Bromid- oder Iodidion, aber auch ein komplexes anorganisches Ion wie Sulfat oder Perchlorat oder auch ein gebräuchliches organisches Anion wie Toluolsulfonat oder Trichloracetat sein. Bevorzugt werden Anionen starker Säuren. Wenn die Hydrazinverbindung an einem Rest mit einer anionischen Gruppe substituiert ist, fällt das Anion gegebenenfalls wegen der Bildung eines inneren Salzes weg.

Bei der Ausarbeitung der Erfindung wurden neue Arylhydrazide gefunden, die durch die allgemeine Formel (II) beschrieben werden.

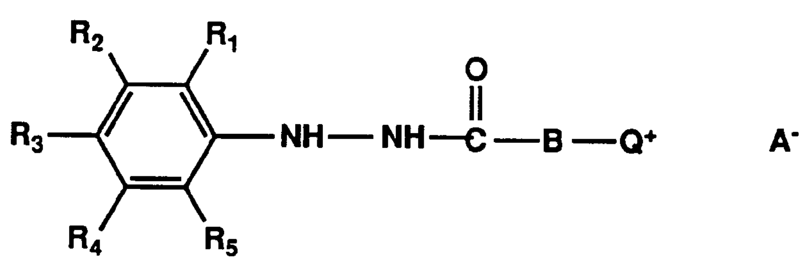

(II)

hierin bedeuten
$R_1$ bis $R_5$ Reste, die gleich oder verschieden sein können, von denen jedoch mindestens einer kein Wasserstoff ist, und die durch Wasserstoff, Halogen, Alkyl, Alkoxy, Hydroxyalkyl, Haloalkyl, Alkylamino, aliphatisches Acylamino mit jeweils 1 bis 20 Kohlenstoffatomen oder Cycloalkyl mit 5 bis 7 Kohlenstoffatomen, Aryl, Aryloxy

oder aromatisches Acylamino mit jeweils 6 bis 10 Kohlenstoffatomen, Aralkyl oder Aralkoxy mit 1 bis 3 Kohlenstoffatomen in der Alkylenkette, einen mit einem ggf. mit einem oder mehreren Alkylresten mit 1 bis 10 Kohlenstoffatomen substituierten Phenoxyrest substituierten aliphatischen Acylaminorest mit 1 bis 4 Kohlenstoffatomen, einen fünf- oder sechsgliedrigen heterocyclischen Ring mit Stickstoff und/oder Schwefel als Heteroatome, der auch an einen Benzolring kondensiert sein kann, oder ein Alkyl- oder Phenylsulfonamidoradikal dargestellt werden, wobei anstelle von zwei Substituenten auch ein gesättigter oder ungesättigter Ring ankondensiert sein kann,

$Q^+$ 3-Alkyl- oder 3-Alkenylimidazolium-1-yl, ggf. weiter substituiert, Chinolinium-yl, Isochinolinium-yl, ggf. substituiert, oder an einem oder mehreren Kohlenstoffatomen substituiertes Pyridinium-yl, ausgenommen Sulfopyridinium-yl, oder, wenn B = -CO-NH- ist, N-Methylpyridinium-3-yl, wobei die Substituenten Alkyl-, Alkenyl-, Aminoalkyl-, Hydroxyalkylreste, Benzyl-, Phenyl-, Phenylmethyl- oder Pyridylreste, Carboxyl-, Carbamid-, Carboxyalkyl-, Cycloalkylthioureidomethyl- oder Hydroxylgruppen, Trialkylammoniummethylgruppen, Amino-, Monoalkylamino- und Dialkylamino- wie auch N-Piperidino- und N-Pyrrolidinogruppen oder auch Chlor sein können, der heterozyklische Ring an einen Benzolring kondensiert sein kann, und

wobei alle Alkylgruppen eines Restes $Q^+$ gleich oder verschieden sein und/oder mit Hydroxyl- oder Sulfosäuregruppen substituiert sein können, jede Alkylgruppe jedoch höchstens 12 Kohlenstoffatome aufweist,

B eine Brücke, die 1 bis 3 Methylengruppen, ein Sauerstoffatom, sowie die Gruppen -CO-NH- oder -NH- enthalten oder, wenn sie nicht unmittelbar an den quaternären Stickstoff anknüpft, auch aus einer einfachen Bindung bestehen kann, wobei die Methylen- und -NH-Gruppen ihrerseits mit Methyl oder Ethyl substituiert sein können.

$A^-$ ein Anion, welches wegfällt, wenn $Q^+$ eine Sulfogruppe oder eine Carboxylgruppe enthält.

Es ist vorteilhaft, wenn die Arylhydrazide am Arylteil oder am Acylteil des Moleküls mit Resten substituiert sind, welche die Adsorption am Silberhalogenid fördern. Solche Reste sind beispielsweise aus den Patentveröffentlichungen DE 26 35 316, DE 26 35 317, DE 28 51 219, DE 29 13 567, DE 29 41 428, DE 29 42 766, DE 29 51 219, DE 29 52 587, EP 23 780 und EP 1 26 000 bekannt.

Besonders bevorzugt werden Arylhydrazide nach der Formel (II), die an einem der Reste $R_1$ bis $R_5$ oder $Q^+$ substituiert sind mit einem Rest der Formel $R_6$-X-CS-X'-, wobei eine der Gruppen X und X durch $NR_7$ und die andere durch $NR_8$, -O- oder -S- und $R_6$, $R_7$ und $R_8$ durch Wasserstoff sowie Alkyl, Cycloalkyl oder Aryl mit bis zu 8 Kohlenstoffatomen dargestellt werden.

Nachstehend werden einige Beispiele für erfindungsgemäße Arylhydrazide nach der Formel (II) angegeben:

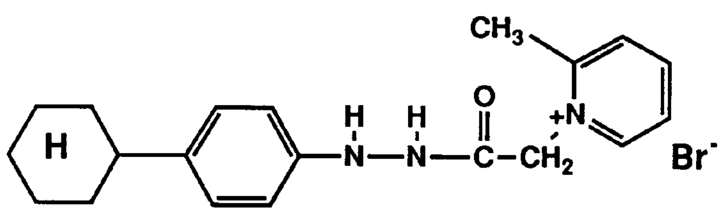

(II-1)

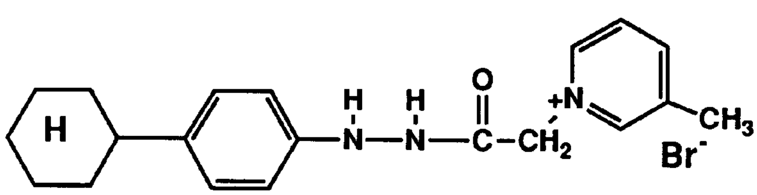

(II-2)

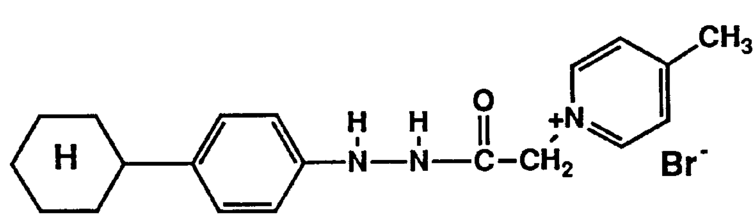

(II-3)

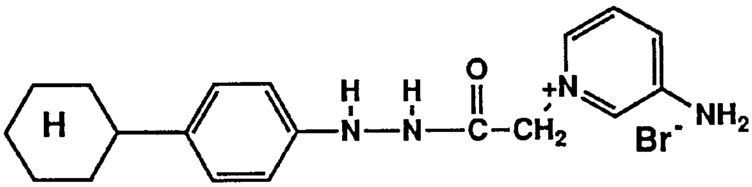

(II-4)

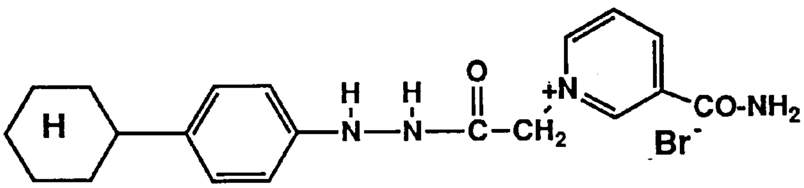

(II-5)

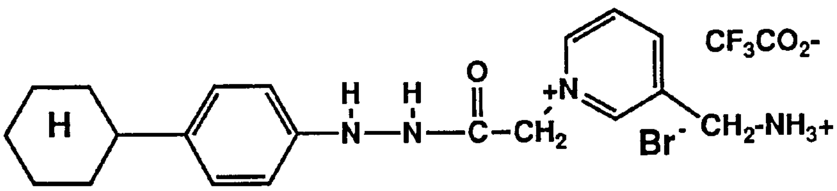

(II-6)

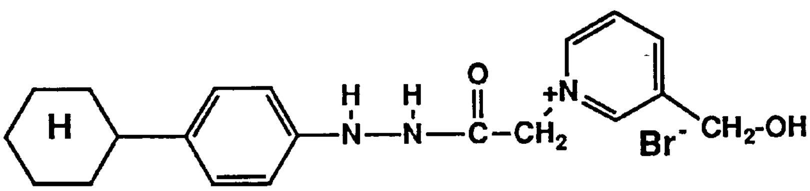

(II-7)

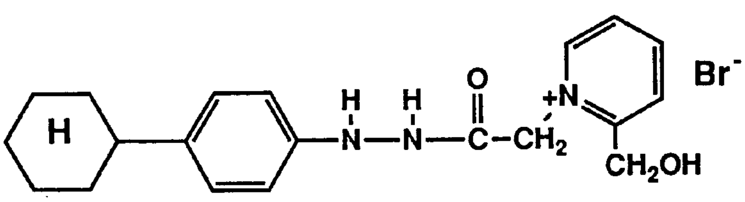

(II-8)

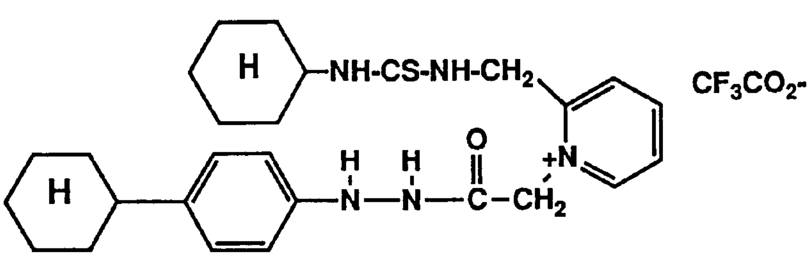

(II-9)

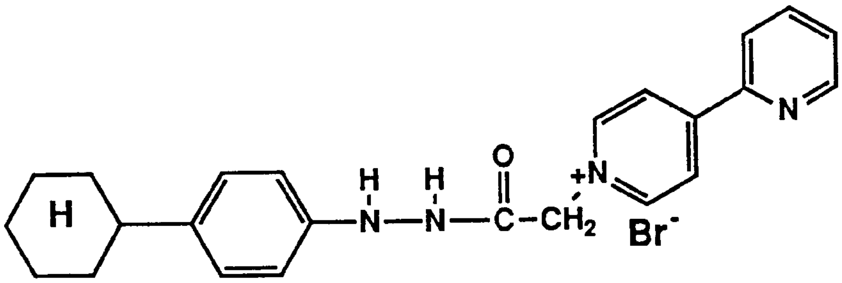

(II-10)

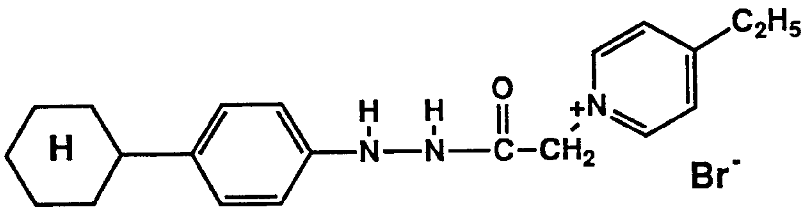

(II-11)

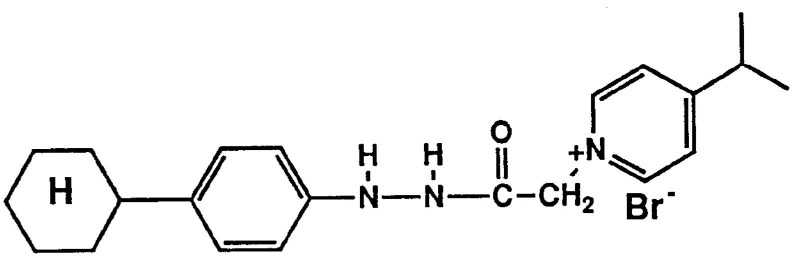

(II-12)

((II-13) frei)

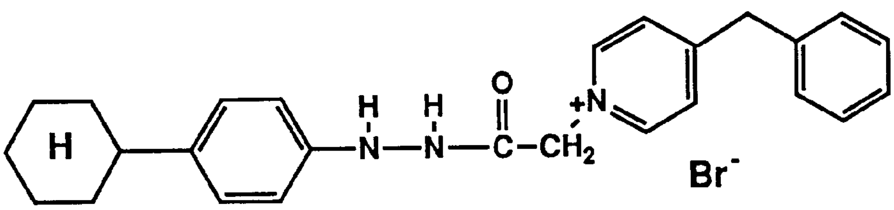

(II-14)

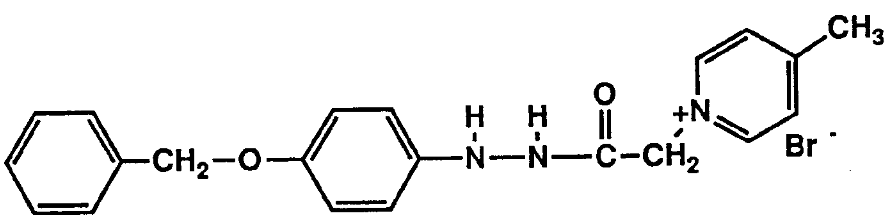

(II-15)

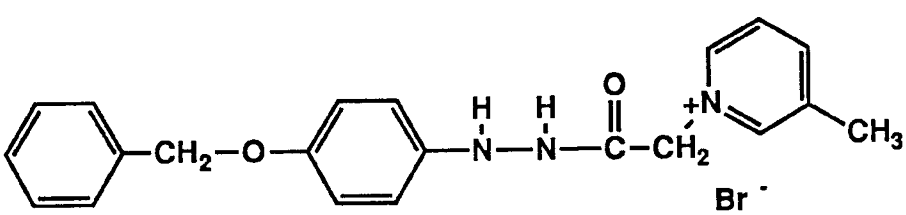

(II-16)

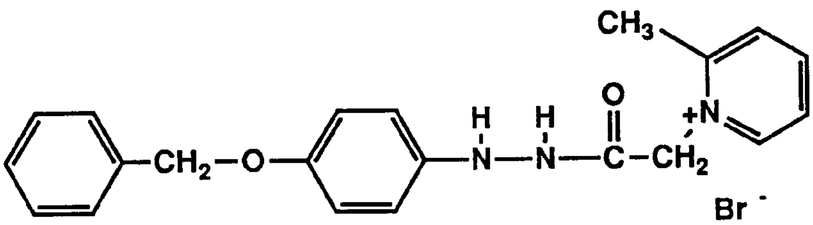

(II-17)

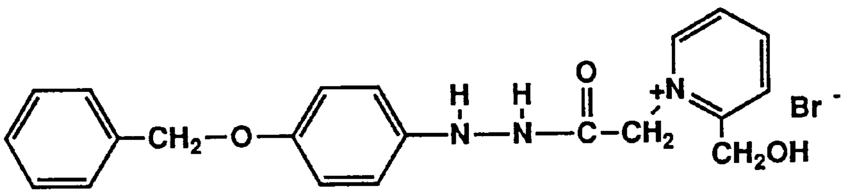

(II-18)

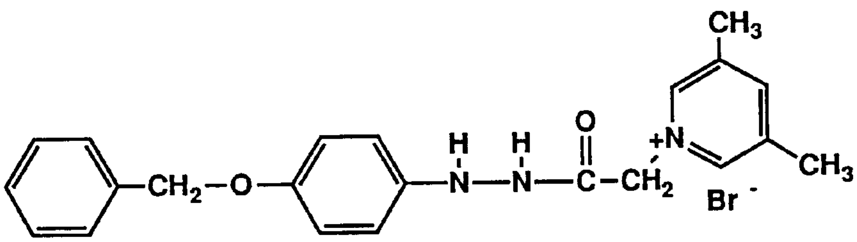

(II-19)

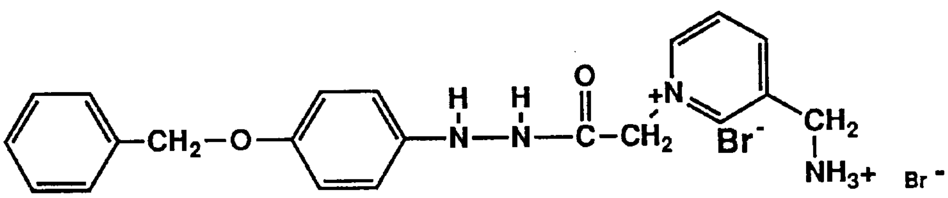

(II-20)

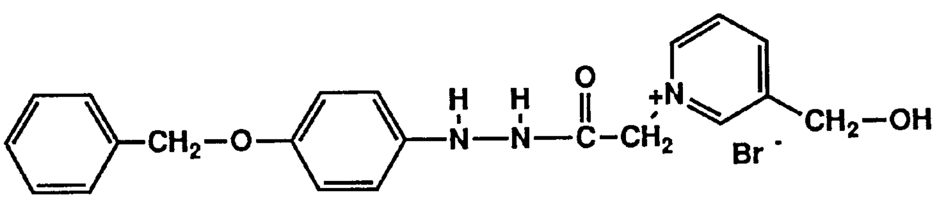

(II-21)

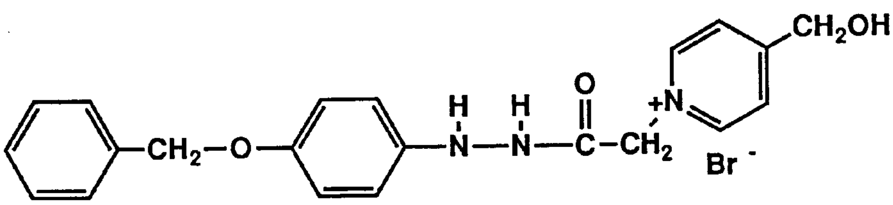

(II-22)

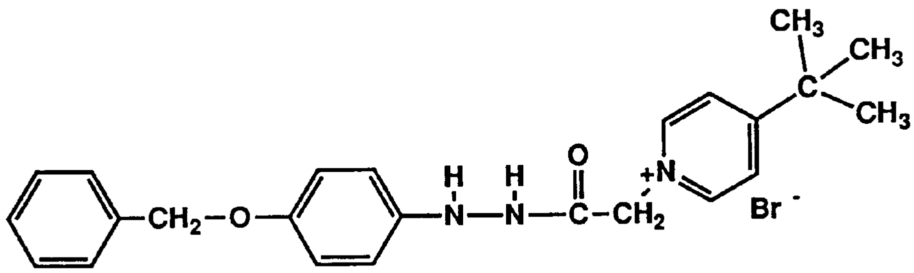

(II-23)

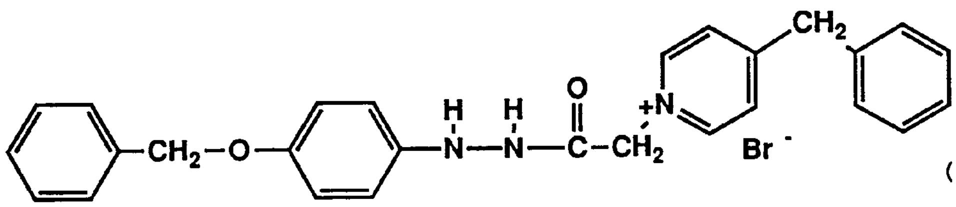

(II-24)

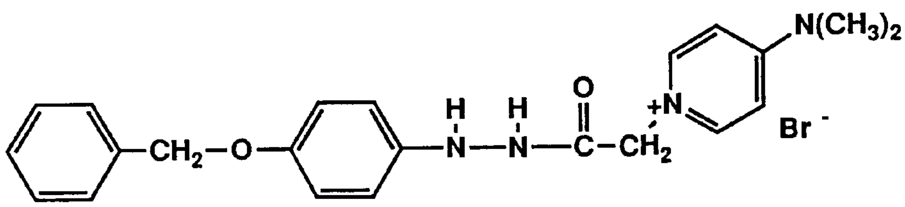

(II-25)

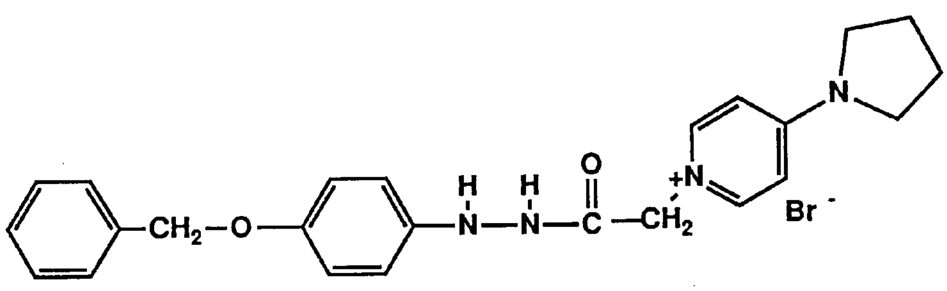

(II-26)

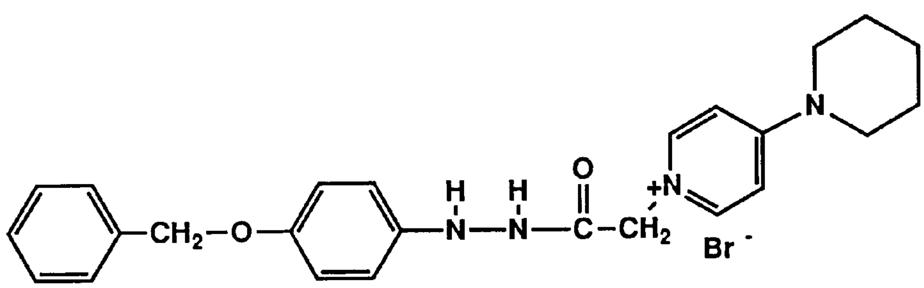

(II-27)

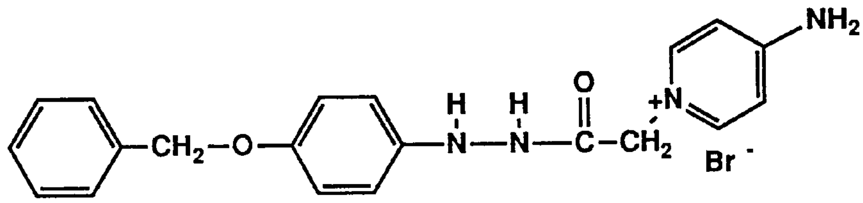

(II-28)

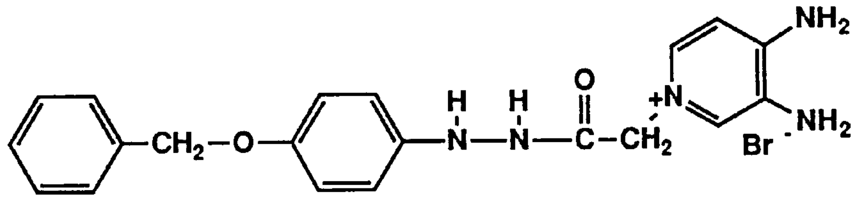

(II-29)

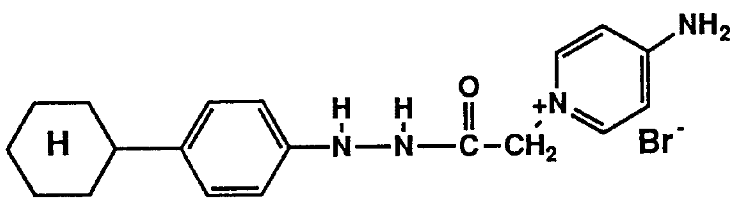

(II-30)

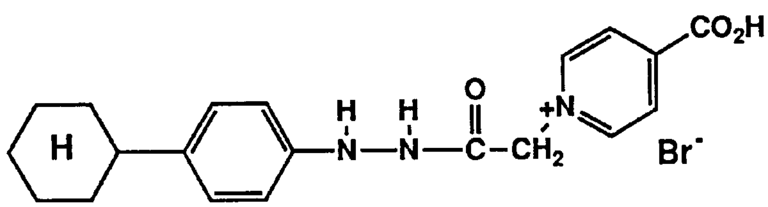

(II-31)

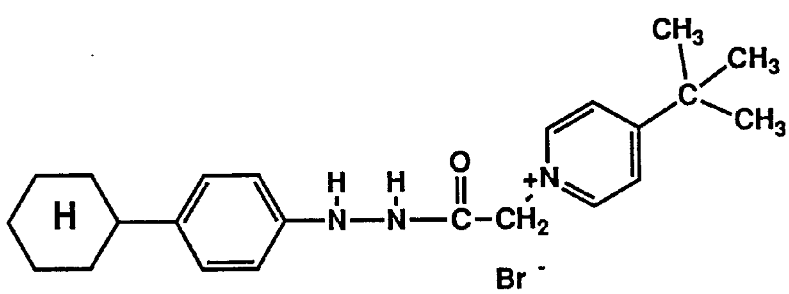

(II-32)

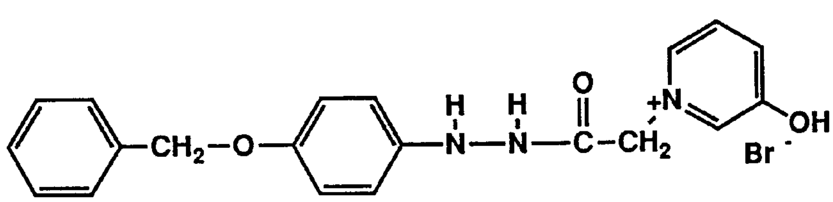

(II-33)

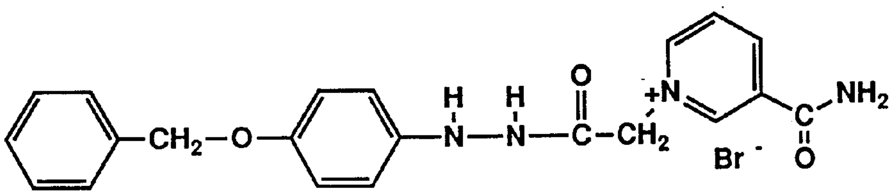

(II-34)

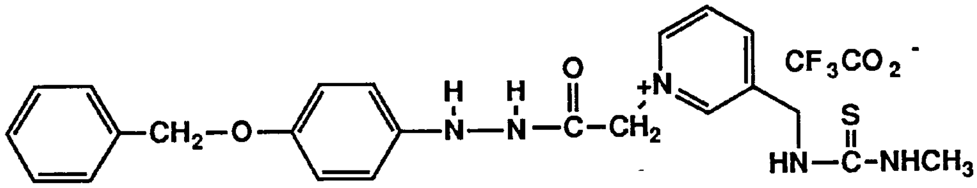

(II-35)

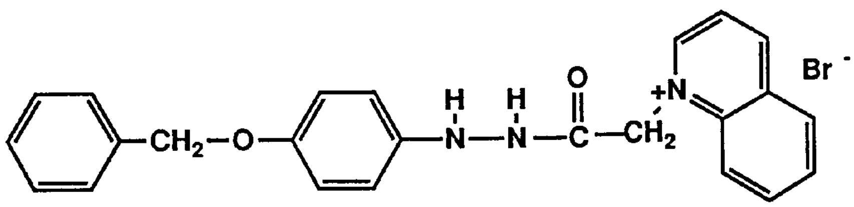

(II-36)

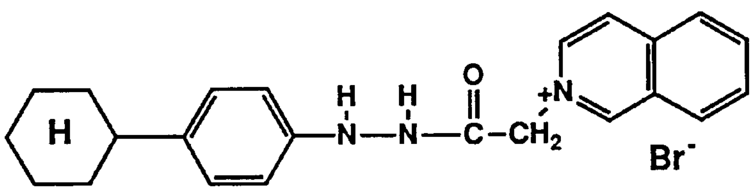

(II-37)

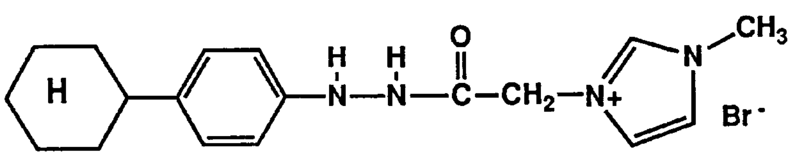

(II-38)

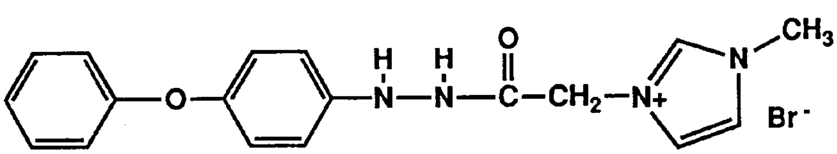

(II-39)

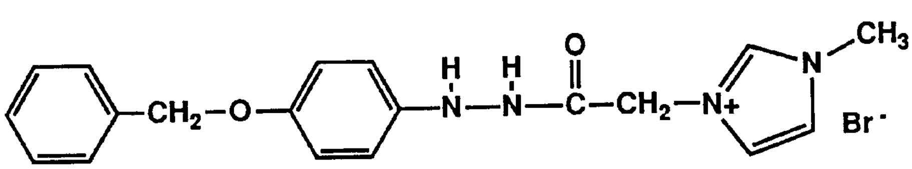

(II-40)

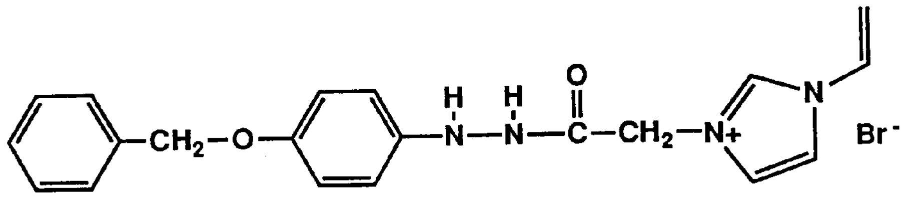

(II-41)

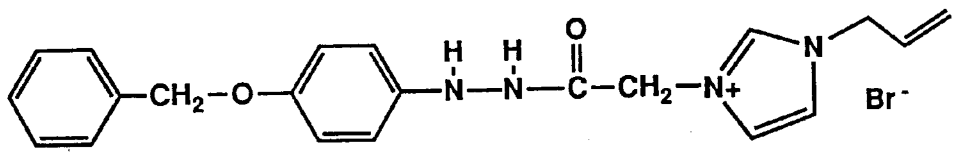

(II-42)

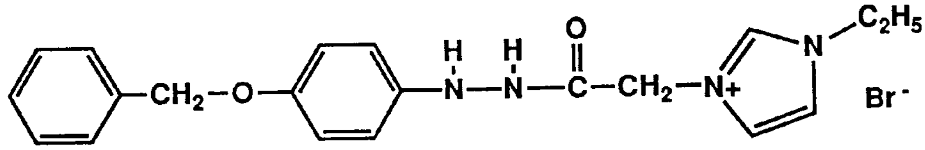

(II-43)

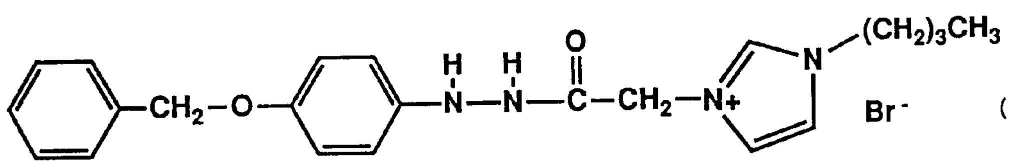

(II-44)

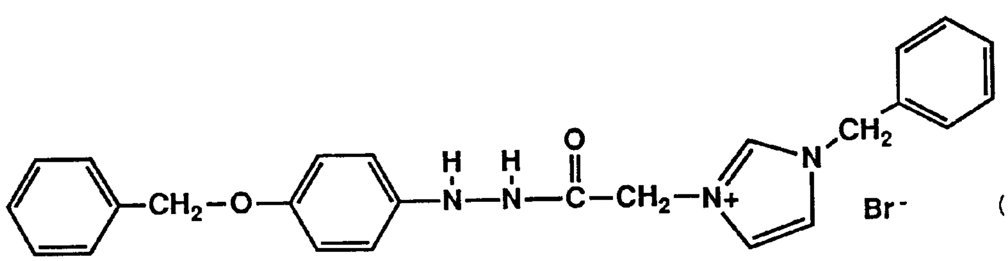

(II-45)

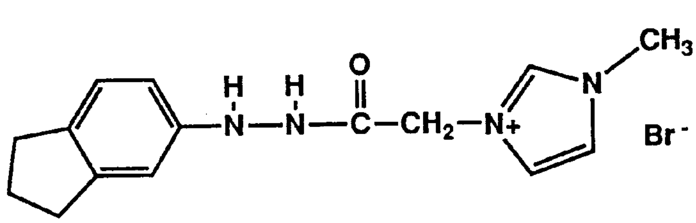

(II-46)

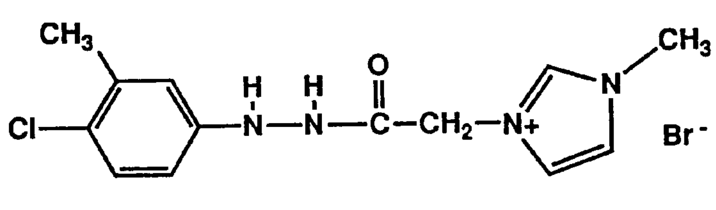

(II-47)

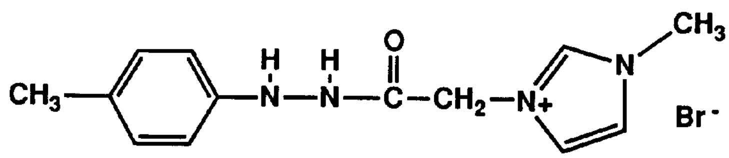

(II-48)

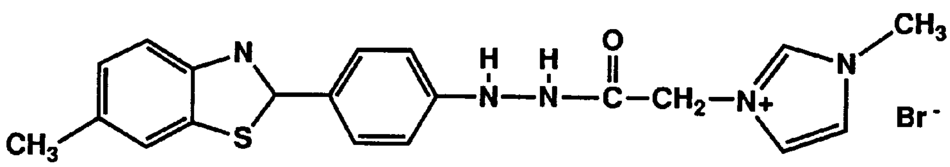

(II-49)

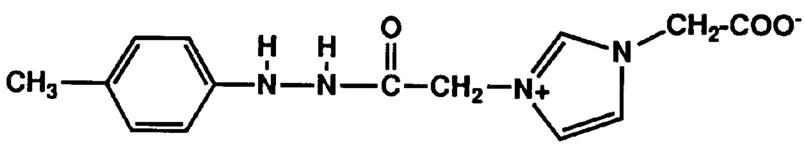

(II-50)

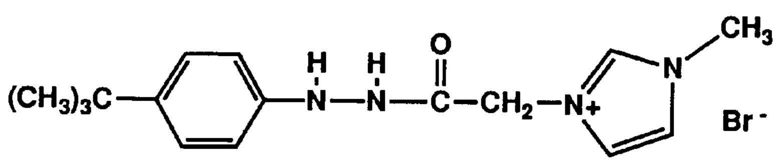

(II-51)

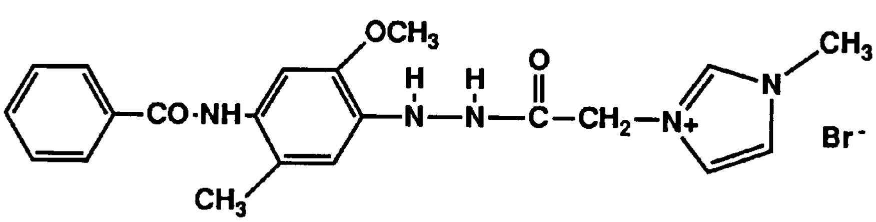

(II-52)

((II-53) und (II-54) frei)

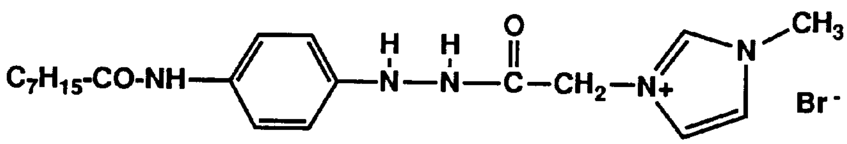

(II-55)

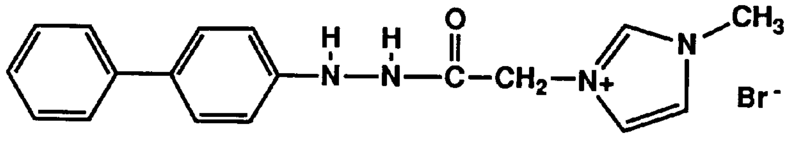

(II-56)

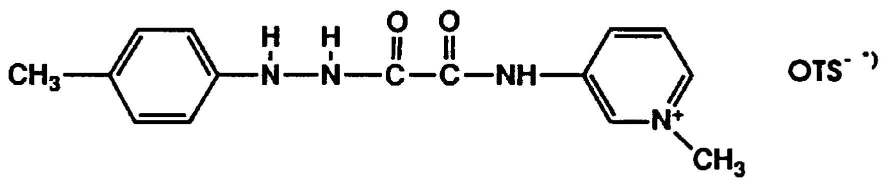

(II-57)

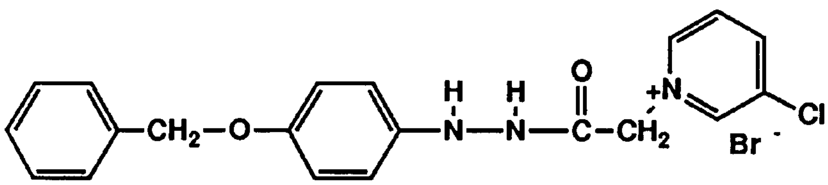

(II-58)

((II-59) frei)

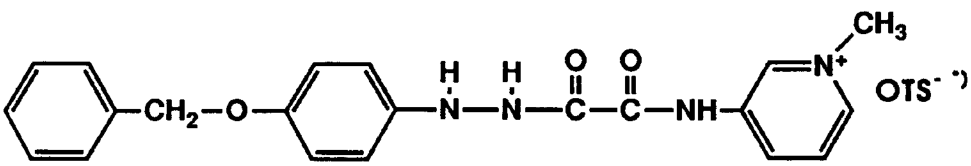

(II-60)

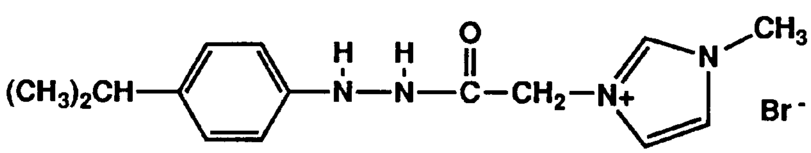

(II-61)

*) p-Toluolsulfonat-Anion

Die erfindungsgemäßen Arylhydrazide können nach verschiedenen Verfahren auf einfache Weise hergestellt werden, beispielsweise aus äquimolaren Mengen des Arylhydrazins, der entsprechenden Carbonsäure und Dicyclohexylcarbodiimid (vgl. Methoden der Organischen Chemie (Houben-Weyl), 4. Aufl., Band X/2, Seite 355). Ein anderer Weg, den Arylrest in das Hydrazid einzubauen, führt über Chinonmonoacylhydrazone bzw. Chinonoximmonoacylhydrazone (vgl. Houben-Weyl, gleicher Band, Seite 233). Eine dritte Möglichkeit besteht in der Hydrazinolyse von Carbonsäureestern (Houben-Weyl, gleicher Band, Seite 360 f.). Weitere Synthesemöglichkeiten sind dem Fachmann bekannt.

Die Herstellung der für die Synthesen benötigten Arylhydrazine selbst kann nach den in Houben-Weyl, Band X/2, S. 169 ff. angegebenen Verfahren erfolgen. Besonders geeignet ist die Diazotierung des entsprechenden Arylamins und die anschließende Reduktion der Diazoniumverbindung mit Zinn-II-chlorid (vergl. Houben-Weyl, Band X/2, S. 203).

Die Synthese der Carbonsäureester mit kationischer Gruppe erfolgt z.B. durch Alkylierung einer tertiären Aminoverbindung mit einem Halogenalkancarbonsäureester in Aceton oder Tetrahydrofuran. Besonders geeignet sind z.B. Bromessigsäureester. Eine andere Möglichkeit besteht darin, einen Carbonsäureester, dessen Carbonsäurerest einen tertiären Stickstoff enthält, mit einem Alkylierungsmittel, wie z.B. Toluolsulfonsäuremethylester, zu quaternisieren.

Eine weitere Synthesemethode ist im Beispiel 1 dargestellt.

Eine besonders bevorzugte Ausführungsform der Erfindung stellen photographische Silberhalogenidmaterialien dar, welche Verbindungen nach der allgemeinen Formel (II) enthalten.

Die lichtempfindlichen Silberhalogenide der erfindungsgemäßen Materialien bestehen aus Silberchlorid, Silberbromid, Silberchlorobromid, Silberbromoiodid oder Silberchlorobromoiodid. Sie können monodispers oder polydispers sein, eine einheitliche Zusammensetzung haben aber auch Körner mit Kern-Schale-Aufbau aufweisen sowie auch Gemische von Körnern verschiedener Zusammensetzung und Korngrößenverteilung sein. Sie werden unter Verwendung eines hydrophilen kolloidalen Bindemittels, bevorzugt Gelatine, hergestellt. Methoden zur Herstellung geeigneter lichtempfindlicher Silberhalogenidemulsionen sind dem Fachmann bekannt und beispielsweise in der Research Disclosure 178 043, Kapitel I und II zusammengefaßt.

Bevorzugt für das erfindungsgemäße Material werden Silberhalogenidemulsionen, die durch kontrollierten Doppelstrahleinlauf hergestellt werden, eine kubische Kornform haben und deren Chloridanteil weniger als 50 Molprozent beträgt.

Die Korngröße der Emulsionen richtet sich nach der erforderlichen Empfindlichkeit und kann zwischen 0,1 und 0,7 µm Kantenlänge betragen, der bevorzugte Bereich liegt zwischen 0,15 und 0,30 µm Kantenlänge. Bei der Emulsionsherstellung können Edelmetallsalze, besonders Salze von Rhodium oder Iridium zur Verbesserung der photographischen Eigenschaften in den üblichen Mengen anwesend sein.

Die Emulsionen werden bevorzugt chemisch sensibilisiert. Geeignete Verfahren sind die Schwefel-, die Reduktions- und die Edelmetallsensibilisierung, die auch in Kombination angewendet werden können. Für letztere können beispielsweise Iridiumverbindungen benutzt werden.

Die Emulsionen können mit üblichen Sensibilisierungsfarbstoffen spektral sensibilisiert werden.

Die Emulsionen können auch übliche Antischleiermittel enthalten. Bevorzugt sind ggf. substituiertes Benztriazol, 5-Nitroindazol und Quecksilberchlorid. Diese Mittel können zu jedem Zeitpunkt bei der Emulsionsherstellung zugesetzt werden oder in einer Hilfsschicht des photographischen Materials enthalten sein. Zur Verbesserung der photographischen Eigenschaften kann der Emulsion vor oder nach der chemischen Reifung ein Jodid in einer Menge von etwa 1 mmol je Mol Silber zugesetzt werden.

Die Emulsionen können auch bekannte Polymerdispersionen enthalten, durch die beispielsweise die Dimensionsstabilität des photographischen Materials verbessert wird. Es handelt sich dabei in der Regel um Latices hydrophober Polymere in wäßriger Matrix. Beispiele für geeignete Polymerdispersionen sind in der Research Disclosure 176 043, Kapitel IX B (Dezember 1978) genannt.

Die lichtempfindlichen Schichten der photographischen Materialien können mit einem bekannten Mittel gehärtet sein. Dieses Härtemittel kann der Emulsion zugesetzt oder über eine Hilfsschicht beispielsweise eine äußere Schutzschicht, eingebracht werden. Ein bevorzugtes Härtungsmittel ist Hydroxydichlorotriazin.

Das photographische Material kann weitere Zusätze, die für die Erzeugung bestimmter Eigenschaften bekannt und üblich sind, enthalten. Solche Mittel sind zum Beispiel in der Research Disclosure 176 043 in den Kapiteln V (Aufheller), XI (Beschichtungshilfsmittel), XII (Weichmacher und Gleitmittel) und XVI (Mattierungsmittel) aufgeführt.

Der Gelatinegehalt der Emulsionen liegt im allgemeinen zwischen 50 und 200 g je Mol Silber; bevorzugt wird der Bereich zwischen 70 und 150 g je Mol Silber.

Die erfindungsgemäßen Arylhydrazide werden bevorzugt der Emulsion inkorporiert, können aber auch in einer mit der Emulsionsschicht in Berührung stehenden Hilfsschicht enthalten sein. Man setzt beispielsweise eine Lösung des Arylhydrazides einer der Gießlösungen zu. Die Zugabe zur Emulsion erfolgt ggf. bevorzugt nach der chemischen Reifung, kann aber auch zu einem anderen Zeitpunkt stattfinden. Ein geeignetes Lösungsmittel für die erfindungsgemäßen Arylhydrazide ist zum Beispiel Ethanol. Die Konzentration der Verbindungen im Film kann über einen weiten Bereich variiert werden und richtet sich neben der Wirksamkeit der Verbindung auch nach den dem Fachmann bekannten Abhängigkeiten der infektiösen Entwicklung von der weiteren Zusammensetzung des Filmes, z.B. dem Bindemittelgehalt und der Bindemittelzusammensetzung, der Halogenidzusammensetzung und der Korngröße der Emulsion, dem Grad der chemischen Reifung der Emulsion sowie der Art und der Menge der Stabilisierung. Eine genaue Abstimmung der Menge mit den genannten Parametern ist dem Fachmann ohne weiteres möglich. Die Konzentration der Verbindungen kann im Bereich zwischen $10^{-5}$ mol/mol Ag bis $5x10^{-2}$ mol/mol Ag liegen, bevorzugt ist der Bereich zwischen $5x10^{-4}$ und $10^{-2}$ mol/mol Ag.

Für die Verarbeitung der erfindungsgemäßen Materialien werden Entwicklerlösungen verwendet, die bevorzugt Dihydroxybenzole wie Hydrochinon als Entwicklersubstanz enthalten. Daneben können sie weitere, auch superadditiv wirkende Entwicklersubstanzen wie 1-Phenylpyrazolidinon oder N-Methyl-p-aminophenol sowie bekannte Antischleiermittel enthalten. Der Sulfitgehalt liegt bevorzugt über 0,15 mol/l. Die Entwicklung wird bevorzugt in Gegenwart weiterer kontraststeigender Mittel, wie z.B. Alkanolamine oder sekundärer aliphatischer oder aromatischer Alkohole, durchgeführt. Die Entwicklertemperatur liegt zwischen 15 und 50° C,

bevorzugt zwischen 30 und 45° C. Die Entwicklerlösung weist einen pH-Wert zwischen 9 und 12,5 auf, wobei der Bereich zwischen 10 und 11,5 bevorzugt wird. Je nach der Entwicklertemperatur kann die Entwicklungszeit 10 bis 500 s betragen.

Fixage, Wässerung und Trocknung der erfindungsgemäßen Materialien können nach bekannten und in der Praxis eingeführten Verfahren erfolgen.

Die erfindungsgemäßen photographischen Silberhalogenidmaterialien lassen sich bereits bei relativ niedrigen pH-Werten und kurzen Entwicklungszeiten zu ultrasteiler Gradation und hervorragender Punktqualität entwickeln. Sie zeigen geringen Schleier und geringe Neigung zur Bildung der dem Fachmann als "Pfeffer" bekannten schwarzen Flecken in nicht bzw. wenig belichteten Bereichen. Der Einfluß des Entwickler-pH-Wertes auf die Entwicklungsgeschwindigkeit und die Empfindlichkeit ist insbesondere im Bereich um pH 11 gering, so daß sich im Betrieb unvermeidliche geringfügige pH-Schwankungen nicht merklich auf das photographische Ergebnis auswirken.

Die erfindungsgemäßen Arylhydrazide zeigen gegenüber den aus dem Stand der Technik bekannten Hydrazinverbindungen, insbesondere gegenüber den Formylhydraziden mit vergleichbarer chemischer Struktur, eine höhere Wirksamkeit als Nucleirungsmittel. Sie können daher in geringeren Mengen eingesetzt werden. Ihre Herstellung ist auf einfache Weise aus leicht zugänglichen Ausgangsstoffen möglich.

Da die erfindungsgemäßen Materialien bei der Entwicklung nicht so hohe pH-Werte benötigen wie Materialien nach dem Stand der Technik ergeben sich Vorteile hinsichtlich der Regenerierungsraten, der Entsorgung verbrauchter Lösungen sowie der Korrosionsbeständigkeit der Entwicklungsgeräte.

Das Anwendungsgebiet der erfindungsgemäßen Materialien ist die Reproduktionstechnik, insbesondere die Herstellung von Rasterbildern aus Halbtonbildern auf herkömmlichem oder auch elektronischem Wege, die Wiedergabe von Strichbildern und Fotomasken für gedruckte Schaltungen oder andere Produkte der Fotofabrikation sowie die Herstellung von Druckvorlagen mittels der Fotosatztechnik. Die erfindungsgemäßen Arylhydrazide können bevorzugt mit lichtempfindlichen Silberhalogeniden angewendet werden.

Obwohl die Erfindung auf Arylhydrazide enthaltende photographische Silberhalogenidmaterialien gerichtet ist, soll ein Verfahren nicht ausgeschlossen werden, bei dem Arylhydrazide auch in der Entwicklerlösung enthalten sind.

Beispiel 1

Herstellung von 1-(p-Tolyl)-5-(methylpyridinium-3-yl)semioxamazidtosylat (Verbindung II-57)

A. 1-Methyl-3-methoxalylamidopyridiniumtosylat

12,26 g (0,1 mol) Oxalsäuremethylesterchlorid, gelöst in 100 ml THF, werden unter Rühren und Eisbadkühlung langsam mit einer Lösung von 9,42 g (0,1 mol) 3-Aminopyridin in 150 ml THF versetzt. Sofort bei der Zugabe fällt ein weißer Feststoff aus. Nach Beendigung der Zugabe wird 1 Stunde nachgerührt und die Mischung dabei auf Raumtemperatur gebracht. Dann wird die Mischung mit 18,2 ml einer 5,5 m methanolischen Natriummethanolat-Lösung versetzt. Nach der Zugabe wird 2 Stunden unter Rückfluß gekocht und anschließend vom ausgefallenen Kochsalz abfiltriert. Das Filtrat wird zur Alkylierung des Zwischenprodukts 3-Methoxalylaminopyridin mit 28 g p-Toluolsulfonsäuremethylester versetzt und 8 Stunden unter Rückfluß gekocht. Die Reaktionsmischung wird dann über Nacht bei Raumtemperatur stehen gelassen, wobei sich ein voluminöser Kristallbrei abscheidet. Der ausgefallene Feststoff wird durch Filtration abgetrennt, mit Aceton gewaschen und anschließend getrocknet. Man erhält 26,5 g (ca. 72% d. Th.) 1-Methyl-3-methoxalylamidopyridiniumtosylat, das ohne weitere Reinigung eingesetzt wird.

B. 1-(p-Tolyl)-5-(methylpyridinium-3-yl)-semioxamazidtosylat

7,95 g (0,05 Mol) Tolylhydrazinhydrochlorid, gelöst in 50 ml Methanol, werden mit Hilfe von Natriummethanolat in die freie Base überführt. Das ausgefallene Kochsalz wird abfiltriert, zum Filtrat werden danach 18,5 g (ca. 0,05 Mol) 1-Methyl-3-methoxalylamidopyridiniumtosylat, gelöst in 50 ml Methanol zugegeben. Die Lösung wird 16 Stunden unter Rückfluß gekocht und anschließend im Gefrierschrank stehen gelassen. Dabei fällt ein gelbes Kristallpulver aus, das abfiltriert und mit Ether gewaschen wird. Durch Zugabe von Ether zum Filtrat der Reaktionslösung fällt eine zweite Fraktion des gelben Kristallpulvers aus, die ebenfalls isoliert und mit der ersten Fraktion vereinigt wird. Das so erhaltene Rohprodukt wird aus Methanol umkristallisiert. Man erhält so 9 g (ca. 40%) 1-(p-Tolyl)-5-(methylpyridinium-3-yl) semioxamazidtosylat als gelbes Pulver (Fp. 200-203° C).

In den Anwendungsbeispielen wurden folgende Vergleichssubstanzen aus dem Stand der Technik verwendet:

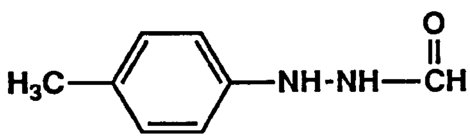

(V-1)

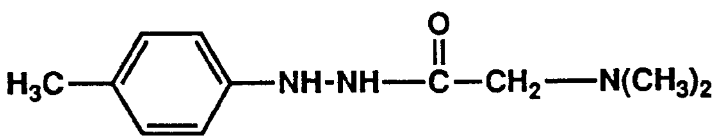

(V-2)

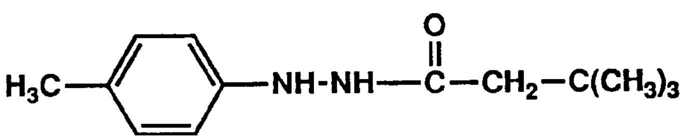

(V-3)

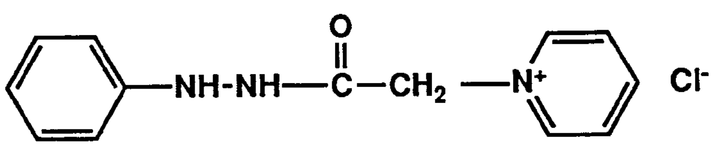

(V-4)

Die Mengenangaben der erfindungsgemäßen und der Vergleichssubstanzen sind stets in mmol je mol Silber als Silberhalogenid.

Beispiel 2

(Anwendungsbeispiel)

Eine Silberbromidemulsion mit kubischen Körnern von 0,25 μm mittlerer Kantenlänge wurde durch pAg-kontrollierten Doppelstrahleinlauf hergestellt. Die Emulsion wurde gewaschen und in Gegenwart von 0,16 mmol Natriumthiosulfat je Mol Silberhalogenid sensibilisiert. Danach wurden ihr übliche Mengen Benzotriazol und 5-Nitroindazol als Antischleiermittel, ein Sensibilisierungsfarbstoff für den grünen Spektralbereich, $2{,}3 \times 10^{-3}$ mol Kaliumjodid pro mol Silber, eine Acrylatpolymerdispersion sowie übliche Beschichtungshilfsmittel zugesetzt. Die Emulsion enthielt 80 g Gelatine pro mol Silber. Gleiche Teile dieser Grundemulsion wurden mit Lösungen der in Tabelle 1 genannten Verbindungen in Ethanol versetzt und auf einen mit einer Lichthofschutzschicht versehenen Polyethylenterephthalat-Schichtträger aufgezogen. Gleichzeitig wurde noch eine Gelatineschutzschicht (1 g/$m^2$ Trockengewicht), die auch ein Härtungsmittel enthielt, aufgetragen. Die so hergestellten Versuchsfilme enthielten 4,4 g Silber je $m^2$. Die Filmproben wurden mit Weißlicht durch eine Vorlage im Kontakt belichtet, die aus einem Halbtonkeil und aus einem mit einem Kontaktraster unterlegten Halbtonkeil bestand. Anschließend wurden die Filme in einer Entwicklungsmaschine (Dürr Graphica) mit Kodak Ultratec Entwickler, dessen pH-Wert vorher durch Zugabe von Schwefelsäure auf 10,8 eingestellt worden war, bei 38°C 30 s entwickelt.

An den verarbeiteten Filmproben wurden folgende Bewertungskriterien bestimmt:

- Dichte von Schleier und Unterlage (Dmin).
- Höchste Dichte (Dmax).
- Relative Empfindlichkeit (S, als - 10 x lg (I x t) bei Dichte 3,0).
- Gradation zwischen den Dichten 1,0 und 3,0 (Gamma).
- Punktqualität (PQ, von 1 - schlechteste bis 10 - beste).

Die Ergebnisse sind in Tabelle 1 zusammengestellt.

Aus ihnen geht hervor, daß die erfindungsgemäßen Verbindungen ultrasteile Gradation und gute Punktqualität schon bei pH-Werten unterhalb von 11 ergeben.

Tabelle 1

| Probe Nr. | Verbindung Nr. | Menge | Dmin | Dmax | S | Gamma | PQ |
|---|---|---|---|---|---|---|---|
| 1 | Kein Zusatz | | 0,04 | 4,20 | 4,0 | 4,5 | 4 |
| 2 | V-1 | 2 | 0,04 | 4,10 | 5,7 | 5,3 | 4 |
| 3 | V-1 | 4 | 0,05 | 4,80 | 6,8 | 6,1 | 4 |
| 4 | V-1 | 8 | 0,05 | 4,60 | 8,1 | 7,4 | 4 |
| 5 | V-2 | 4 | 0,04 | 4,4 | 4,5 | 5,4 | 4 |
| 6 | V-2 | 8 | 0,05 | 4,4 | 4,7 | 6,0 | 4 |
| 7 | V-3 | 4 | 0,04 | 4,10 | 3,90 | 4,7 | 4 |
| 8 | V-3 | 8 | 0,04 | 4,20 | 3,80 | 4,7 | 4 |
| 9 | V-4 | 0,65 | 0,05 | 4,9 | 7,0 | 5,8 | 4 |
| 10 | V-4 | 1,30 | 0,05 | 5,0 | 8,0 | 6,5 | 4 |
| 11 | V-4 | 4,0 | 0,06 | 5,0 | 9,3 | 8,2 | 4 |
| 12 | II-1 | 0,65 | 0,04 | 4,9 | 9,4 | 18 | 9-10 |
| 13 | II-2 | 0,65 | 0,04 | 4,9 | 9,4 | 17 | 9-10 |
| 14 | II-3 | 0,65 | 0,04 | 5,1 | 10,0 | 20 | 10 |
| 15 | II-7 | 1,3 | 0,04 | 5,1 | 9,2 | 14 | 8-9 |
| 16 | II-8 | 1,3 | 0,04 | 4,7 | 9,0 | 14 | 8-9 |
| 17 | II-32 | 1,3 | 0,04 | 4,8 | 9,2 | 16 | 10 |
| 18 | II-40 | 0,65 | 0,04 | 5,2 | 11,5 | 23 | 10 |
| 19 | II-41 | 0,65 | 0,4 | 5,2 | 11,3 | 21 | 10 |
| 20 | II-34 | 1,3 | 0,04 | 4,7 | 9,8 | 12 | 9 |
| 21 | II-48 | 0,65 | 0,05 | 5,0 | 11,4 | 20 | 7 |
| 24 | II-57 | 1,3 | 0,05 | 5,0 | 9,6 | 18 | 7 |

Beispiel 3

Eine kubische Silberbromidemulsion wurde wie in Beispiel 2 beschrieben hergestellt und sensibilisiert. Danach wurden ihr übliche Mengen Benzotriazol als Antischleiermittel, ein Sensibilisierungsfarbstoff für den grünen Spektralbereich, 2,3 x $10^{-3}$ mol Kaliumjodid pro mol Silber, eine Polyethylendispersion und übliche Beschichtungshilfsmittel zugesetzt. Die Emulsion enthielt 80 g Gelatine pro mol Silber.

Gleiche Teile dieser Emulsion wurden dann mit Lösungen der in Tabelle 2 genannten Verbindungen in Ethanol versetzt. Aus den Emulsionen wurden dann wie in Beispiel 2 beschrieben, Versuchsfilme hergestellt.

Proben dieser Filme wurden, wie in Beispiel 2 beschrieben, belichtet und anschließend in einer Entwicklungsmaschine mit einem Entwickler folgender Zusammensetzung bei 36° C 40 s lang entwickelt.

Entwicklerrezept:

| | Zusätze in g/l |
|---|---|
| Wasser | 600 |
| KOH | 30 |
| $K_2S_2O_5$ | 66 |
| EDTA | 3 |
| $Na_2CO_3$ $H_2O$ | 48 |
| KBr | 3 |
| Benzotriazol | 0,5 |
| Phenylmercaptotetrazol | 0,05 |
| Hydrochinon | 25 |
| N-Methyl-p-aminophenolsulfat | 1,5 |
| Diethylaminopropandiol | 25 |
| Wasser auf | 1 l |
| pH auf 10,9 bei 20° C | . |

Die Auswertung erfolgte nach den in Beispiel 2 genannten sensitometrischen Kriterien; jedoch wurde in dem gerasterten Verlaufskeil die Punktqualität bei 3 verschiedenen Punktgrößen mit 80facher Vergrößerung visuell bestimmt. Folgende Bereiche wurden zur Beurteilung herausgezogen:

Hochlichter: um 95%,
Mittelton: um 50%,
Tiefen: um 10%,

Beurteilungskriterien waren die Kontur- und Kantenschärfe der Punkte sowie der Zwischenpunktschleier, der besonders bei den Hochlichtern sichtbar wird.

Die Ergebnisse sind in der Tabelle 2 zusammengestellt.

Die Ergebnisse zeigen erneut, daß ultrasteile Gradation und gute Punktqualität bereits durch sehr geringe Konzentration der erfindungsgemäßen Verbindungen schon bei niedrigen pH-Werten erreicht werden.

| Probe Nr. | Verbindung Nr. | Menge | Dmin | Dmax | S | Gamma | PQ 10% | PQ 50% | PQ 90% |
|---|---|---|---|---|---|---|---|---|---|
| 1 | II-3 | 1,2 | 0,05 | 5,2 | 10,2 | 16 | 9 | 8-9 | 6 |
| 2 | II-15 | 0,4 | 0,05 | 5,3 | 10,8 | 20 | 10 | 10 | 7-8 |
| 3 | II-16 | 0,4 | 0,04 | 5,2 | 10,8 | 18 | 10 | 10 | 8 |
| 4 | II-17 | 0,4 | 0,04 | 5,3 | 10,7 | 18 | 10 | 10 | 7 |
| 5 | II-22 | 0,4 | 0,05 | 5,0 | 9,7 | 12 | 9 | 9 | 7 |
| 6 | II-23 | 0,4 | 0,05 | 5,2 | 10,0 | 14 | 9 | 9-10 | 7-8 |
| 7 | II-28 | 0,4 | 0,04 | 5,4 | 10,9 | 23 | 10 | 10 | 9 |
| 8 | II-25 | 0,4 | 0,04 | 5,4 | 10,8 | 24 | 10 | 10 | 8 |
| 9 | II-26 | 0,4 | 0,04 | 5,0 | 9,0 | 12 | 9 | 9-10 | 7 |
| 10 | II-26 | 0,9 | 0,04 | 5,0 | 9,5 | 22 | 10 | 10 | 8 |
| 11 | II-27 | 0,4 | 0,05 | 5,1 | 9,2 | 12 | 9 | 9 | 7 |
| 12 | II-27 | 0,6 | 0,04 | 5,1 | 9,4 | 20 | 10 | 10 | 9 |
| 13 | II-29 | 0,4 | 0,04 | 5,2 | 10,0 | 15 | 10 | 10 | 7-8 |
| 14 | II-30 | 1,2 | 0,04 | 5,0 | 9,5 | 18 | 9 | 9 | 7 |
| 15 | II-35 | 0,6 | 0,05 | 5,0 | 7,0 | 20 | 9 | 8-9 | 6 |
| 16 | II-40 | 0,4 | 0,04 | 5,3 | 10,8 | 25 | 10 | 10 | 9 |
| 17 | II-41 | 0,4 | 0,04 | 5,2 | 10,7 | 22 | 10 | 10 | 9 |
| 18 | II-42 | 0,4 | 0,04 | 5,3 | 10,2 | 22 | 10 | 10 | 9 |
| 19 | II-44 | 0,4 | 0,04 | 5,2 | 11,1 | 20 | 10 | 10 | 9 |
| 20 | II-45 | 0,4 | 0,04 | 5,2 | 9,3 | 20 | 10 | 10 | 9 |
| 21 | II-58 | 0,4 | 0,04 | 5,0 | 8,0 | 8 | 8 | 8 | 6 |
| 22 | II-36 | 0,6 | 0,05 | 5,0 | 7,8 | 15 | 9 | 8-9 | 6 |
| 23 | V-4 | 3,6 | 0,05 | 4,6 | 7,0 | 5,0 | 3 | 4 | 3 |
| 24 | Keine | | 0,04 | 4,3 | 4,5 | 4,8 | 3 | 4 | 3 |

## Patentansprüche

1. Photographische Silberhalogenidmaterialien für die Erzeugung von Bildern mit ultrasteiler Gradation, die Arylhydrazide der allgemeinen Formel (I) enthalten;

$$Ar - NR - NR^1 - G - X^+ A^- \qquad (I)$$

worin
Ar eine substituierte Phenylgruppe oder eine andere substituierte oder unsubstituierte Arylgruppe,
G die Gruppe CO, SO, $SO_2$, Phosphonyl, Phosphoryl oder $C{=}NR^2$,
$X^+$ einen Rest, der eine kationische Gruppe mit einem quaternierten Stickstoffatom enthält,
R, $R^1$, $R^2$ Wasserstoff, Alkyl oder Alkylsulfinyl mit 1 bis 6 Kohlenstoffatomen,
$A^-$ ein Anion bedeuten,
dadurch gekennzeichnet, daß
das quaternierte Stickstoffatom in eine substituierte oder unsubstituierte Imidazolium-, Imidazolinium-,

Isochinolinium-, Chinoliniumgruppe oder in eine an einem oder mehreren Kohlenstoffatomen substituierte Pyridiniumgruppe, ausgenommen Sulfoethylpyridinium, eingebunden ist.

**2.** Photographische Silberhalogenidmaterialien nach Anspruch 1, dadurch gekennzeichnet, daß sie Arylhydrazide der Formel (II) enthalten,

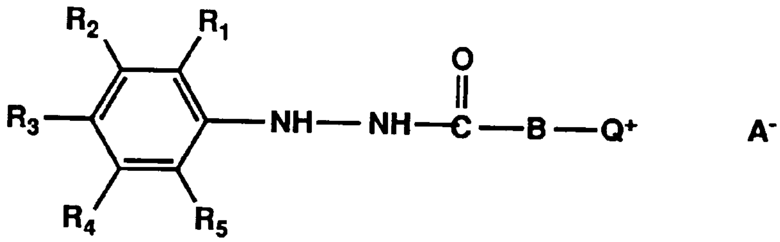

(II)

hierin bedeuten
$R_1$ bis $R_5$ Reste, die gleich oder verschieden sein können, von denen jedoch mindestens einer kein Wasserstoff ist, und die durch Wasserstoff, Halogen, Alkyl, Alkoxy, Hydroxyalkyl, Haloalkyl, Alkylamino, aliphatisches Acylamino mit jeweils 1 bis 20 Kohlenstoffatomen oder Cycloalkyl mit 5 bis 7 Kohlenstoffatomen, Aryl, Aryloxy oder aromatisches Acylamino mit jeweils 6 bis 10 Kohlenstoffatomen, Aralkyl oder Aralkoxy mit 1 bis 3 Kohlenstoffatomen in der Alkylenkette, einen mit einem ggf. mit einem oder mehreren Alkylresten mit 1 bis 10 Kohlenstoffatomen substituierten Phenoxyrest substituierten aliphatischen Acylaminorest mit 1 bis 4 Kohlenstoffatomen, einen fünf- oder sechsgliedrigen heterocyclischen Ring mit Stickstoff und/oder Schwefel als Heteroatome, der auch an einen Benzolring kondensiert sein kann, oder ein Alkyl- oder Phenylsulfonamidoradikal dargestellt werden, wobei anstelle von zwei Substituenten auch ein gesättigter oder ungesättigter Ring ankondensiert sein kann,
$Q^+$ 3-Alkyl- oder 3-Alkenylimidazolium-1-yl, ggf. weiter substituiert, Chinolinium-yl, Isochinolinium-yl, ggf. substituiert, oder an einem oder mehreren Kohlenstoffatomen substituiertes Pyridinium-yl, ausgenommen Sulfopyridinium-yl, oder, wenn B = -CO-NH- ist, N-Methylpyridinium-3-yl, wobei die Substituenten Alkyl-, Alkenyl-, Aminoalkyl-, Hydroxyalkylreste, Benzyl-, Phenyl-, Phenylmethyl- oder Pyridylreste, Carboxyl-, Carbamid-, Carboxyalkyl-, Cycloalkylthioureidomethyl- oder Hydroxylgruppen, Trialkylammoniummethylgruppen, Amino-, Monoalkylamino- und Dialkylamino- wie auch N-Piperidino- und N-Pyrrolidinogruppen oder auch Chlor sein können, der heterozyklische Ring an einen Benzolring kondensiert sein kann, und
wobei alle Alkylgruppen eines Restes $Q^+$ gleich oder verschieden sein und/oder mit Hydroxyl- oder Sulfosäuregruppen substituiert sein können, jede Alkylgruppe jedoch höchstens 12 Kohlenstoffatome aufweist, B eine Brücke, die 1 bis 3 Methylengruppen, ein Sauerstoffatom, sowie die Gruppen -CO-NH- oder -NH-enthalten oder, wenn sie nicht unmittelbar an den quaternären Stickstoff anknüpft, auch aus einer einfachen Bindung bestehen kann, wobei die Methylen- und -NH-Gruppen ihrerseits mit Methyl oder Ethyl substituiert sein können,
$A^-$ ein Anion, welches wegfällt, wenn $Q^+$ eine Sulfogruppe oder eine Carboxylgruppe enthält.

**3.** Photographische Silberhalogenidmaterialien nach Anspruch 2, dadurch gekennzeichnet, daß das in ihnen enthaltene Arylhydrazid nach Formel (II) an einem der Reste $R_1$ bis $R_5$ oder $Q^+$ substituiert ist mit einem Rest der Formel $R_6$-X-CS-X'-, wobei eine der Gruppen X und X' durch $NR_7$ und die andere durch $NR_8$, -O- oder -S- und $R_6$, $R_7$ und $R_6$ durch Wasserstoff sowie Alkyl, Cycloalkyl oder Aryl mit bis zu 8 Kohlenstoffatomen dargestellt werden.

**4.** Arylhydrazide der Formel (II)

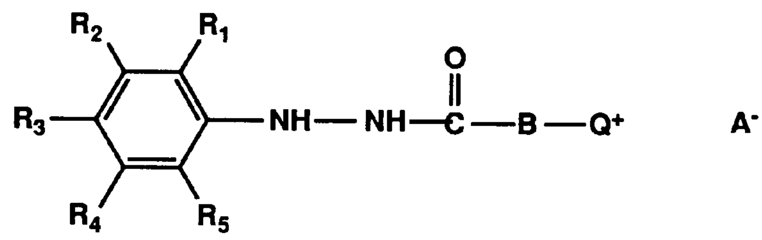

(II)

hierin bedeuten

$R_1$ bis $R_5$ Reste, die gleich oder verschieden sein können, von denen jedoch mindestens einer kein Wasserstoff ist, und die durch Wasserstoff, Halogen, Alkyl, Alkoxy,

Hydroxyalkyl, Haloalkyl, Alkylamino, aliphatisches Acylamino mit jeweils 1 bis 20 Kohlenstoffatomen oder Cycloalkyl mit 5 bis 7 Kohlenstoffatomen, Aryl, Aryloxy oder aromatisches Acylamino mit jeweils 6 bis 10 Kohlenstoffatomen, Aralkyl oder Aralkoxy mit 1 bis 3 Kohlenstoffatomen in der Alkylenkette, einen mit einem ggf. mit einem oder mehreren Alkylresten mit 1 bis 10 Kohlenstoffatomen substituierten Phenoxyrest substituierten aliphatischen Acylaminorest mit 1 bis 4 Kohlenstoffatomen, einen fünf- oder sechsgliedrigen heterocyclischen Ring mit Stickstoff und/oder Schwefel als Heteroatome, der auch an einen Benzolring kondensiert sein kann, oder ein Alkyl- oder Phenylsulfonamidoradikal dargestellt werden, wobei anstelle von zwei Substituenten auch ein gesättigter oder ungesättigter Ring ankondensiert sein kann,

$Q^+$ 3-Alkyl- oder 3-Alkenylimidazolium-1-yl, ggf. weiter substituiert, Chinolinium-yl, Isochinolinium-yl, ggf. substituiert, oder an einem oder mehreren Kohlenstoffatomen substituiertes Pyridinium-yl, ausgenommen Sulfopyridinium-yl, oder, wenn B = -CO-NH- ist, N-Methylpyridinium-3-yl, wobei die Substituenten Alkyl-, Alkenyl-, Aminoalkyl-, Hydroxyalkylreste, Benzyl-, Phenyl-, Phenylmethyl- oder Pyridylreste, Carboxyl-, Carbamid-, Carboxyalkyl-, Cycloalkylthioureidomethyl- oder Hydroxylgruppen, Trialkylammoniummethylgruppen, Amino-, Monoalkylamino- und Dialkylamino- wie auch N-Piperidino- und N-Pyrrolidinogruppen oder auch Chlor sein können, der heterozyklische Ring an einen Benzolring kondensiert sein kann, und

wobei alle Alkylgruppen eines Restes $Q^+$ gleich oder verschieden sein und/oder mit Hydroxyl- oder Sulfosäuregruppen substituiert sein können, jede Alkylgruppe jedoch höchstens 12 Kohlenstoffatome aufweist, B eine Brücke, die 1 bis 3 Methylengruppen, ein Sauerstoffatom, sowie die Gruppen -CO-NH- oder -NH-enthalten oder, wenn sie nicht unmittelbar an den quaternären Stickstoff anknüpft, auch aus einer einfachen Bindung bestehen kann, wobei die Methylen- und -NH-Gruppen ihrerseits mit Methyl oder Ethyl substituiert sein können.

$A^-$ ein Anion, welches wegfällt, wenn $Q^+$ eine Sulfogruppe oder eine Carboxylgruppe enthält.

## Claims

1. Photographic silver halide materials for generation of ultrahigh contrast images, containing aryl hydrazides of the general formula (I)

$$Ar - NR - NR^1 - G - X^+ A^- \qquad (I)$$

wherein

Ar is a substituted phenyl or another substituted or unsubstituted aryl group,

G is CO, SO, $SO_2$, phosphonyl, phosphoryl, or $C{=}NR^2$,

$X^+$ is a radical containing a cationic group with a quaternized nitrogen atom,

R, $R^1$, $R^2$ are hydrogen, alkyl, or alkylsulfinyl with 1 to 6 carbon atoms,

$A^-$ is an anion,

characterized in that

the quaternized nitrogen atom is included in a substituted or unsubstituted imidazolium, imidazolinium, isoquinolinium, quinolinium group or in a pyridinium group substituted on one or more carbon atoms, with the exception of sulfoethylpyridinium.

2. Photographic silver halide materials according to claim 1,

characterized in that

they contain aryl hydrazides of formula (II)

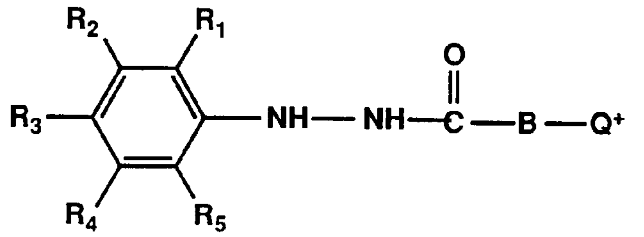

A- (II)

wherein

$R_1$ to $R_5$ are radicals, which can be equal or different, at least one of them is, however, not hydrogen, and which are represented by hydrogen, halogen, alkyl, alkoxy, hydroxyalkyl, haloalkyl, alkylamino, aliphatic acylamino, each having 1 to 20 carbon atoms, or cycloalkyl having 5 to 7 carbon atoms, aryl, aryloxy, or aromatic acylamino, each having 6 to 10 carbon atoms, aralkyl or aralkoxy having 1 to 3 carbon atoms in the alkylen chain, an aliphatic acylamino radical having 1 to 4 carbon atoms and being substituted with a phenoxy radical, the latter being optionally substituted with one or more alkyl radicals each having 1 to 10 carbon atoms, a five- or six-membered heterocyclic ring having nitrogen and/or sulfur as hetero atoms, which can also be condensed to a benzene ring, or an alkyl or phenyl sulfonamido radical, wherein in place of two substituents a saturated or unsaturated ring can be condensed to,

$Q^+$ is 3-alkyl- or 3-alkenylimidazolium-1-yl, optionally further substituted, quinolinium-yl, isoquinolinium-yl, optionally substituted, or pyridinium-yl optionally substituted at one or more of its carbon atoms, with the exception of sulfopyridinium-yl, or, if B = -CO-NH-, is N-methylpyridinium-3-yl, wherein the substituents can be alkyl, alkenyl, aminoalkyl, or hydroxyalkyl radicals, benzyl, phenyl, phenylmethyl, or pyridyl radicals, carboxyl, carbamido, carboxyalkyl, cyclolalkylthioureidomethyl or hydroxyl groups, trialkylammoniummethyl groups, amino, monoalkylamino and dialkylamino as well as N-piperidino and N-pyrrolidino groups or also chlorine, the heterocyclic ring can be condensed to a benzene ring, and

wherein all alkyl groups of a radical $Q^+$ can be equal or different and/or can be substituted by hydroxyl groups or sulfonic acid groups, each alkyl group having at most 12 carbon atoms,

B is a link which may contain 1 to 3 methylen groups, an oxygen atom, as well as the groups -CO-NH- or -NH-, or which, if it is not immediately bound to the quaternary nitrogen, may consist of a single bond, wherein the methylen and NH groups can be substituted by methyl or ethyl,

$A^-$ is an anion, which is omitted if $Q^+$ contains a sulfo or a carboxy group.

**3.** Photographic silver halide materials according to claim 2, characterized in that the aryl hydrazide of formula (II) contained therein is substituted at one of the radicals $R_1$ to $R_5$ or $Q^+$ with a radical of formula $R_6$-X-CS-X′-, wherein one of the groups X and X′ is represented by $NR_7$ and the other by $NR_5$, -O- or -S-, and $R_5$, $R_7$, and $R_8$ are represented by hydrogen as well as alkyl, cycloalkyl or aryl having at most 8 carbon atoms.

**4.** Aryl hydrazides of formula (II)

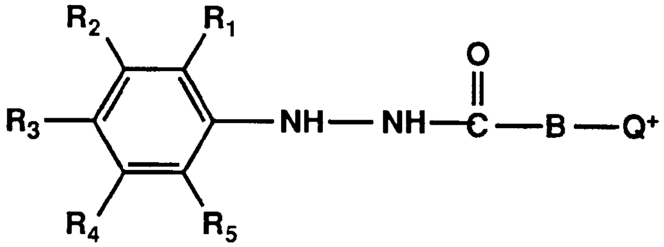

A- (II)

wherein

$R_1$ to $R_5$ are radicals, which can be equal or different, at least one of them is, however, not hydrogen, and which are represented by hydrogen, halogen, alkyl, alkoxy, hydroxyalkyl, haloalkyl, alkylamino, aliphatic acylamino, each having 1 to 20 carbon atoms, or cycloalkyl having 5 to 7 carbon atoms, aryl, aryloxy, or aromatic acylamino, each having 6 to 10 carbon atoms, aralkyl

or aralkoxy having 1 to 3 carbon atoms in the alkylen chain, an aliphatic acylamino radical having 1 to 4 carbon atoms and being substituted with a phenoxy radical, the latter being optionally substituted with one or more alkyl radicals each having 1 to 10 carbon atoms, a five- or six-membered heterocyclic ring having nitrogen and/or sulfur as hetero atoms, which can also be condensed to a benzene ring, or an alkyl or phenyl sulfonamido radical, wherein in place of two substituents a saturated or unsaturated ring can be condensed to,

$Q^+$ is 3-alkyl- or 3-alkenylimidazolium-1-yl, optionally further substituted, quinolinium-yl, isoquinolinium-yl, optionally substituted, or pyridinium-yl optionally substituted at one or more of its carbon atoms, with the exception of sulfopyridinium-yl, or, if B = -CO-NH-, is N-methylpyridinium-3-yl, wherein the substituents can be alkyl, alkenyl, aminoalkyl, or hydroxyalkyl radicals, benzyl, phenyl, phenylmethyl, or pyridyl radicals, carboxyl, carbamido, carboxyalkyl, cyclolalkylthioureidomethyl or hydroxyl groups, trialkylammoniummethyl groups, amino, monoalkylamino and dialkylamino as well as N-piperidino and N-pyrrolidino groups or also chlorine, the heterocyclic ring can be condensed to a benzene ring, and

wherein all alkyl groups of a radical $Q^+$ can be equal or different and/or can be substituted by hydroxyl groups or sulfonic acid groups, each alkyl group having at most 12 carbon atoms,

B is a link which may contain 1 to 3 methylen groups, an oxygen atom, as well as the groups -CO-NH- or -NH-, or which, if it is not immediately bound to the quaternary nitrogen, may consist of a single bond, wherein the methylen and NH groups can be substituted by methyl or ethyl,

$A^-$ is an anion, which is omitted if $Q^+$ contains a sulfo or a carboxy group.

## Revendications

1. Matériaux photographiques à base d'halogénure d'argent, pour la formation d'images à gradation ultracontrastée, qui contiennent des arylhydrazides de formule générale (I):

$$Ar - Nr - NR^1 - G - X^+ A^- \qquad (I)$$

dans laquelle:

| | |
|---|---|
| Ar | signifie un groupe phényle substitué ou un autre groupe aryle substitué ou non substitué; |
| G | représente les groupes CO, SO, $SO_2$, phosphonyle, phosphoryle ou $C{=}NR^2$; |
| $X^+$ | représente un radical qui contient un groupe cationique renfermant un atome d'azote quaternisé; |
| R, $R^1$ et $R^2$ | représentent un atome d'hydrogène, un radical alkyle ou alkylsulfinyle renfermant de 1 à 6 atomes de carbone; |
| $A^-$ | signifie un anion; |

caractérisés en ce que l'atome d'azote quaternisé est incorporé dans un groupe imidazolium, imidazolinium, isoquinolinium ou quinolinium, substitué ou non substitué, ou dans un groupe pyridinium substitué sur un seul ou plusieurs atomes de carbone, à l'exception du groupe sulfoéthylpyridinium.

2. Matériaux photographiques à base d'halogénure d'argent selon la revendication 1, caractérisé en ce qu'ils contiennent des arylhydrazides de formule (II):

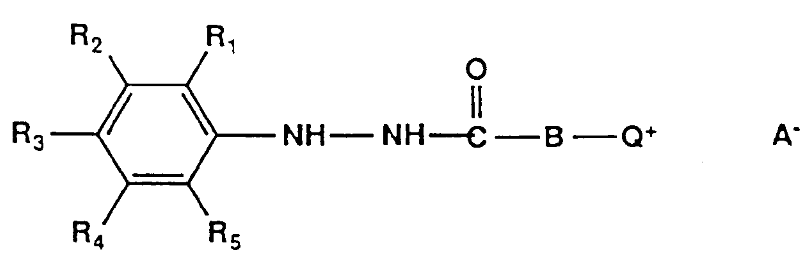

(II)

dans laquelle:

$R_1$ à $R_5$ sont des radicaux qui peuvent être identiques ou différents, au moins l'un d'entre eux cependant n'étant pas un atome d'hydrogène, et qui sont constitués par un atome d'hydrogène, un atome d'halogène, un radical alkyle, alcoxy, hydroxyalkyle, haloalkyle, alkylamino, acylamino aliphatique renfermant chaque fois de 1 à 20 atomes de carbone ou cycloalkyle renfermant de 5 à 7 atomes de carbone, aryle, aryloxy ou acylamino aromatique

renfermant chaque fois de 6 à 10 atomes de carbone, aralkyle ou aralcoxy dont la chaîne alkylène renferme de 1 à 3 atomes de carbone, un radical acylamino aliphatique renfermant de 1 à 4 atomes de carbone, éventuellement substitué par un radical phénoxy luimême substitué par un ou plusieurs radicaux alkyle renfermant de 1 à 10 atomes de carbone, un cycle hétérocyclique à 5 ou 6 éléments renfermant de l'azote et/ou du soufre comme hétéroatomes, qui peut également être condensé sur un cycle dibenzène, ou un radical alkyle ou phényle sulfonamido, dans lesquels, au lieu de deux substituants, on peut aussi avoir un cycle saturé ou non saturé formé par condensation un cycle saturé ou non saturé;

$Q^+$ signifie un radical 3-alkyl- ou 3-alkénylimidazolium-1-yle-, éventuellement davantage substitué, quinolinium-yle, isoquinolinium-yle, le cas échéant substitué, ou pyridinium-yle substitué sur un ou plusieurs atomes de carbone, à l'exception du sulfopyridinium-yle ou, lorsque B représente CO-NH-, N-méthylpyridinium-3-yle, les substituants pouvant être des radicaux alkyle, alkényle, aminoalkyle, hydroxyalkyle, benzyle, phényle, phénylméthyle ou pyridyle, carboxyle, carbamide, carboxyalkyle, cycloalkylthiouréeidométhyle ou hydroxyle, des groupes trialkylammoniumméthyle, amino-, monoalkylamino et dialkylamino ainsi que également N-piperidino et N-pyrrolidino ou également un atome de chlore, et le cycle hétérocyclique peut être condensé sur un cycle benzène, et tous les groupes alkyle, identiques ou différents, pouvant être des radicaux $Q^+$ et/ou être substitués par des groupes hydroxyle ou acide sulfonique, chaque groupe alkyle comportant cependant au maximum 12 atomes de carbone;

B signifie un pont qui peut contenir de 1 à 3 groupes méthylène, un atome d'hydrogène ainsi que les groupes -CO-NH- ou NH ou bien, lorsqu'il ne se rattache pas directement à l'atome d'azote quaternaire, il peut également être constitué d'une liaison simple, les groupes méthylène et les groupes NH pour leur part pouvant être substitués par le radical méthyle ou éthyle;

$A^-$ signifie un anion qui n'est pas présent lorsque $Q^+$ contient un groupe sulfo ou un groupe carboxyle.

3. Matériaux photographiques à base d'halogénure d'argent selon la revendication 2, caractérisés en ce que le groupe arylhydrazide de formule (II) qu'ils contiennent est substitué sur l'un des radicaux $R_1$ à $R_5$ ou $Q^+$ par un radical de formule $R_5$-X-CS-X', l'un des groupes X et X' étant représenté par $NR_7$ et l'autre par $RN_8$, O ou S, et $R_6$, $R_7$ et $R_8$ par un atome d'hydrogène ainsi que par un radical alkyle, cycloalkyle ou aryle renfermant jusqu'à 8 atomes de carbone.

4. Arylhydrazides de formule (II):

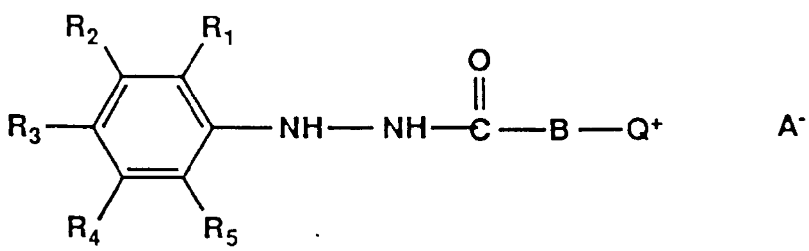

(II)

dans laquelle:

$R_1$ à $R_5$ sont des radicaux qui peuvent être identiques ou différents, au moins l'un d'entre eux cependant n'étant pas un atome d'hydrogène et qui sont constitués par un atome d'hydrogène, un atome d'halogène, un radical alkyle, alcoxy, hydroxyalkyle, haloalkyle, alkylamino, acylamino aliphatique renfermant chaque fois de 1 à 20 atomes de cabone ou cycloalkyle renfermant de 5 à 7 atomes de carbone, aryle, aryloxy ou acylamino aromatique renfermant chaque fois de 6 à 10 atomes de carbone, aralkyle ou aralcoxy dont la chaîne alkylène renferme de 1 à 3 atomes de carbone, un radical acylamino aliphatique renfermant de 1 à 4 atomes de carbone, substitué par un radical phénoxy lui-même substitué, le cas échéant, par un ou plusieurs radicaux alkyle renfermant de 1 à 10 atomes de carbone, un cycle hétérocyclique à 5 ou 6 éléments renfermant de l'azote et/ou du soufre

comme hétéroatomes, qui peut également être condensé sur un cycle dibenzène, ou un radical alkyl- ou phénylsulfonamido, dans lequel, au lieu de deux substituants, peut être appliqué par condensation un cycle saturé ou non saturé peut être appliqué par condensation;

$Q^+$ signifie un radical 3-alkyl- ou 3-alkénylimidazolium-1-yle, éventuellement encore substitué, quinolinium-yle, isoquinolinium-yle, le cas échéant substitué, ou pyridinium-yle substitué sur un ou plusieurs atomes de carbone, à l'exception du sulfopyridinium-yle ou, lorsque B représente -CO-NH-, N-méthylpyridinium-3-yle, dans laquelle les substituants peuvent être des radicaux alkyle, alkényle, aminoalkyle, hydroxyalkyle, des radicaux benzyle, phényle, phénylméthyle ou pyridyle, des groupes carboxyle, carbamide, carboxyalkyle, cycloalkylthiouréeidométhyle ou hydroxyle, des groupes trialkylammoniumméthyle, des groupes amino-, monoalkylamino et dialkylamino ainsi que également N-piperidino et N-pyrrolidino ou également un atome de chlore et le cycle hétérocyclique pouvant être condensé sur un cycle benzène, et tous les groupes alkyle, identiques ou différents, pouvant être des radicaux $Q^+$ et/ou être substitués par des groupes hydroxyle ou acide sulfonique, chaque groupe alkyle comportant cependant au maximum 12 atomes de carbone;

B signifie un pont qui peut contenir de 1 à 3 groupes tous les groupes alkyle d'un radical $Q^+$ pouvant être identiques ou différents et/ou être substitués par des groupes hydroxyles ou des groupes acide sulfonique, chaque groupe alkyle comportant cependant au maximum 12 atomes de carbone;

$A^-$ signifie un anion qui n'est pas présent lorsque $Q^+$ contient un groupe sulfo ou un groupe carboxyle.